# EUROPEAN PATENT APPLICATION

(11) **EP 3 460 469 A1**
(43) Date of publication of application: **27.03.2019**
(21) Application number: 17798767.4
(22) Date of filing: 19.05.2017
(51) Int. Cl.: G01N 33/487

(54) **DETECTION DEVICE AND DETECTION METHOD**

(30) Priority: 19.05.2016 CN 201610333833
(71) Applicant: Quanovate Global Ltd, Grand Cayman, KY1-1104 (KY)
(72) Inventor: QIU, Mengchun, Hangzhou Zhejiang 310018 (CN); LIN, Jijun, Hangzhou Zhejiang 310018 (CN); WANG, Shaoyang, Hangzhou Zhejiang 310018 (CN); YANG, Zheng, CA 94588 (US)
(74) Representative: Mewburn Ellis LLP
(86) International application number: PCT/CN2017/085010
(87) International publication number: WO 2017/198204

(57) **Abstract**

Disclosed herein are a detection device and a detection method. The detection device comprises: a consumable cassette (1) configured to have a detection side (A) provided with a consumable, consumable cassette (1) being provided with a circuit board (3) carrying a test information; and a detection instrument configured to read the test information from the circuit board (3) and perform a detection to the consumable based on the test information to obtain a detection result. Also provided is a detection method comprising: providing the detection device (S1); placing the consumable cassette (1) into the detection instrument (S2); obtaining a test information by the detection instrument (S3); determining, according to the test information, whether the consumable is in compliance with a detection requirement (S4); and performing a detection to the consumable by the detection instrument to obtain a detection result when the consumable is in compliance with the detection requirement (S5). The test information is obtained with lower cost. An accuracy and an efficiency of the detection are improved.

## Description

### TECHNICAL FIELD

The present disclosure is directed to field of medical devices, and more particularly to a detection device and a detection method.

### BACKGROUND

In vitro detection instruments can obtain clinical diagnosis information by detecting human biological samples (for example, blood, body fluids, tissues, etc.) to determine an occurrence of disease or a condition of body function.

In vitro detection instruments can comprise various disposable detection consumables, such as test strips, test strip cassettes, reagents, reagent holding devices and biochips.

Information on a type of the detection consumables is required as a number of detection consumables can be used for in vitro detection. Information on production batch is required as a difference can be found in consumables of same type but different production batches, such that parameters can be obtained to correct a detection result. In addition, information on whether the consumable is expired, reused and containing anti-counterfeiting information is also required for in vitro detection.

In order to obtain test information, a barcode can be provided on the detection consumable and the in vitro detection instrument can scan the barcode to obtain the test information. However, the information provided by barcode can be limited. For example, only simple information such as a product category can be provided by barcode, and no information such as a detection result can be written into the detection consumable. In addition, in order to scan the barcode, a reflection light path is introduced to form a barcode scanning channel, resulting in a complicated structure and increased cost of the in vitro detection instrument.

### SUMMARY

There is a need to provide a detection device and a detection method which is capable of obtaining test information at a low cost.

In an aspect, the present disclosure provides a detection device. The detection device can comprise: a consumable cassette configured to have a detection side provided with a consumable, the consumable cassette being provided with a circuit board carrying a test information; and a detection instrument configured to read the test information from the circuit board and perform a detection to the consumable based on the test information to obtain a detection result.

In some embodiments, the circuit board can comprise a chip disposed on the circuit board for storing the test information; and a first connection end which is electrically connected with the chip.

In some embodiments, the detection instrument can comprise: a detecting unit configured to perform a detection on the consumable; and a reading unit configured to read the test information. The detection instrument can further comprise a slot configured to receive the consumable cassette, and a second connection end connected with the reading unit can be provided in the slot.

In some embodiments, the first connection end can comprise a contact plate covering an outer surface of the detection cassette, and the second connection end comprises a contact point matching the contact plate.

In some embodiments, the first connection end can comprise contact plates which are provided in a matrix, and the second connection end can comprise contact points which are provided in a matrix.

In some embodiments, the detection side can be provided with a detection area through which the consumable is exposed.

In some embodiments, the test information carried in the chip can comprise one or more of an anti-counterfeiting code, a category of detection, a consumable number, a consumable batch number, date of production, expiration date, time of first read/write, number of read/write operations, a limit for number of read-write, a limit for consumable reaction time, a threshold for insufficient sampling volume, name of test line, name of control line, a parameter of signal pre-processing, a threshold for reaction over time failure, a threshold for consumable failure, a calibration curve, a correction parameter, a critical value of result gear level, and an unit of testing.

In some embodiments, the parameter of signal pre-processing can comprise one or more of a center point position of test line, a center point position of control line, a half-peak width and a baseline width.

In some embodiments, the calibration curve can comprises a type of curve and/or a parameter of curve.

In some embodiments, the chip can be a read/write chip, and the chip can be configured to further store a test result.

Another aspect of the disclosure provides a detection method. The detection method can comprise: providing the detection device; placing the consumable cassette into the detection instrument; obtaining the test information by the detection instrument; determining, according to the test information, whether the consumable is in compliance with a detection requirement; and performing a detection to the consumable by the detection instrument to obtain a detection result when the consumable is in compliance with the detection requirement.

In some embodiments, obtaining the test information by the detection instrument can comprise determining whether the consumable cassette is correctly installed. In some embodiments, determining, according to the test information, whether the consumable is in compliance with the detection requirement can comprise reading the test information from the circuit board when the consumable cassette is correctly installed, and determining whether the consumable is in compliance with the detection requirement according to the test information.

In some embodiments, determining, according to the test information, whether the consumable is in compliance with the detection requirement can comprise: determining the consumable is readable when the detection instrument is able to read the test information from the circuit board and write a data into the circuit board; displaying a message indicating that the consumable is not readable and terminating the detection when the consumable is not readable; and performing a further detection when the consumable is readable.

In some embodiments, the test information can comprise an anti-counterfeiting code. In some embodiments, determining, according to the test information, whether the consumable is in compliance with the detection requirement can comprise: determining, according to the anti-counterfeiting code, whether the consumable is a counterfeit; displaying a message indicating that the consumable is a counterfeit and terminating the detection when the consumable is a counterfeit; and performing a further detection when the consumable is not a counterfeit.

In some embodiments, the test information can comprise a category of detection, a consumable number and a consumable batch number. In some embodiments, determining, according to the test information, whether the consumable is in compliance with the detection requirement can comprise: determining, according to the category of detection, the consumable number and the consumable batch number, whether the consumable is a product being recalled; displaying a message indicating that the consumable is a product being recalled and terminating the detection when the consumable is a product being recalled; and performing a further detection when the consumable is not to be a product being recalled.

In some embodiments, the test information can comprise date of production and expiration date. In some embodiments, determining, according to the test information, whether the consumable is in compliance with the detection requirement can comprise: determining, according to the date of production, the expiration date and a current time of the detection instrument, whether the consumable is expired; displaying a message indicating that the consumable is expired and terminating the detection when the consumable is expired; and performing a further detection when the consumable is not expired.

In some embodiments, the test information can comprises a limit for consumable reaction time. In some embodiments, determining, according to the test information, whether the consumable is in compliance with the detection requirement can comprise: determining, by comparing an actual reaction time of the consumable and the limit for consumable reaction time, whether a reaction time of the consumable is beyond limit; displaying a message indicating that the reaction time of the consumable is beyond limit and terminating the detection when the consumable reaction time is beyond limit; and performing a further detection when the consumable reaction time is not beyond limit.

In some embodiments, the detection instrument can be configured to, once the consumable cassette is inserted into the detection instrument, write into the circuit board of the consumable cassette a current system time as an actual reaction start time of the consumable. The actual reaction start time of the consumable can be not deleted or modified. The actual reaction time of the consumable can be determined from the actual reaction start time of the consumable and the current system time.

In some embodiments, the test information can comprise a limit for number of read-write. In some embodiments, determining, according to the test information, whether the consumable is in compliance with the detection requirement can comprises: determining whether the consumable is overused by comparing an actual number of read-write of the consumable cassette with the limit for number of read-write, said actual number of read-write of the consumable cassette being stored in the circuit board of the consumable cassette; displaying a message indicating that the consumable is overused when the consumable is overused; and performing a further detection when the consumable is not overused.

In some embodiments, the actual number of read-write of the consumable cassette is increased by one each time the consumable cassette is inserted into the detection instrument. The actual number of read-write of the consumable cassette cannot be deleted or modified to a smaller number. The further detection can be performed when the actual number of read-write of the consumable cassette is zero.

In some embodiments, the test information can comprise a threshold for insufficient sampling volume. In some embodiments, determining, according to the test information, whether the consumable is in compliance with the detection requirement can comprises: determining whether the sampling volume of the consumable is insufficient by comparing a calculated value of a pre-scanned data of the consumable by the detection instrument with the threshold for insufficient sampling volume; displaying a message indicating that the sampling volume of the consumable is insufficient and terminating the detection when the sampling volume of the consumable is insufficient; and performing a further detection when the sampling volume of the consumable is sufficient.

In some embodiments, the calculated value of the pre-scanned data can be an intensity of a signal. In some instances, the detection instrument can be configured to perform a sampling at one or multiple positions of the detection side of the consumable, and average multiple sampling values to obtain the calculated value of the pre-scanned data. In some instances, the detection instrument can be configured to perform a sampling at multiple positions of the detection side of the consumable, and average multiple sampling values to obtain the calculated value of the pre-scanned data.

In some embodiments, the test information can comprise a threshold for reaction over time failure. In some embodiments, determining, according to the test information, whether the consumable is in compliance with the detection requirement can comprise: determining whether the consumable reaction time is unreasonably long by comparing a calculated value of a pre-scanned data of the consumable by the detection instrument with the threshold for reaction over time failure; displaying a message indicating that the consumable reaction time is unreasonably long and terminating the detection when the consumable reaction time is unreasonably long; and performing a further detection when the consumable reaction time is not unreasonably long.

In some embodiments, the calculated value of the pre-scanned data can be an intensity of a signal. In some instances, the detection instrument can be configured to perform a sampling at one or more positions of the detection side of the consumable, and average multiple sampling values to obtain the calculated value of the pre-scanned data. In some instances, the detection instrument can be configured to perform a sampling at multiple positions of the detection side of the consumable at multiple time points, and average multiple sampling values to obtain the calculated value of the pre-scanned data.

In some embodiments, the test information can comprise a threshold for consumable failure. In some embodiments, determining, according to the test information, whether the consumable is in compliance with the detection requirement can comprise: determining whether the consumable fails by comparing a calculated value of a control line data scanned by the detection instrument with the threshold for consumable failure; displaying a message indicating that the consumable fails and terminating the detection when the consumable fails, and calculating and displaying a measurement result when the consumable does not fail.

In some embodiments, the calculated value of the control line data can be a peak height and/or peak area of a control line. The peak height and/or peak area of the control line can be calculated from a signal intensity of control line measured by the detection instrument in combination with a center point position, a half-peak width and a baseline width of consumable control line contained in the test information.

In some embodiments, the detection result can comprise a single signal or a set of signals. The detection result can be calculated from a test line signal intensity of the consumable measured by the detection instrument in combination with a center point position a half-peak width and a baseline width of consumable test line contained in the test information. A measurement result and a gear level of measurement result of target object can be calculated based on the detection result in combination with a calibration curve, a correction parameter and a critical value of result gear level contained in the test information.

In some embodiments, the circuit can comprise a chip for storing the test information; a first connection end for electrically connecting the chip with an external circuit. The detection device can comprise a detection unit configured to perform a detection on the consumable; a reading unit configured to read the test information. In some instances, the detection instrument can further comprise a slot configured to receive the consumable cassette. A second connection end connected with the reading unit can be provided in the slot. In some instances, determining whether the consumable cassette is correctly installed can comprise determining whether the second connection end is in a connected state.

In some embodiments, performing a detection on the consumable by the detection instrument can comprise obtaining an initial detection result by the detection device, and converting or correcting the initial detection result based on the test information to obtain a final detection result. The test information can comprise one or more of a calibration curve, a correction parameter, or a unit of testing.

In some embodiments, the detection method can further comprise, after performing a detection to the consumable, updating the test information stored in the circuit board of the consumable cassette based on the detection result.

In some embodiments, updating the test information stored in the circuit board of the consumable cassette can comprise increasing a number of read/write in the circuit board.

In some embodiments, updating the test information stored in the circuit board of the consumable cassette can comprise writing the detection result to the circuit board.

Another aspect of the disclosure provides a consumable cassette. The circuit board can comprise a detection side provided with a consumable. The consumable cassette can be provided with a circuit board carrying a test information.

In some embodiments, the test information can comprise one or more of an anti-counterfeiting code, a category of detection, a consumable number, a consumable batch number, date of production, expiration date, time of first read/write, number of read/write operations, a limit for number of read-write, a limit for consumable reaction time, a threshold for insufficient sampling volume, name of test line, name of control line, a parameter of signal pre-processing, a threshold for reaction over time failure, a threshold for consumable failure, a calibration curve, a correction parameter, a critical value of result gear level, and an unit of testing.

In some embodiments, the parameter of signal pre-processing can comprise one or more of a center point position of test line, a center point position of control line, a half-peak width and a baseline width.

In some embodiments, the calibration curve can comprise a type of curve and/or a parameter of curve.

Another aspect of the disclosure provides a detection instrument. The detection instrument can comprise: a reading device configured to read a test information from a consumable cassette, the consumable cassette being provided with a consumable and a circuit board carrying the test information; and one or more processors configured collectively or individually to: determine, according to the test information, whether the consumable is in compliance with a detection requirement; and perform a detecting on the consumable to obtain a detection result when the consumable is in compliance with the detection requirement.

In some embodiments, the one or more processors can be further configured to: determine whether the consumable cassette is correctly installed; read the test information from the circuit board when the consumable cassette is correctly installed; and determine whether the consumable is in compliance with the detection requirement according to the test information.

In some embodiments, the one or more processors can be further configured to: determine the consumable is readable when the test information is able to be read from the circuit board and data is able to be stored into the circuit board; display a message indicating that the consumable is not readable and terminate the detection when the consumable is not readable; and perform a further detection when the consumable is readable.

In some embodiments, the test information can comprise an anti-counterfeiting code. The one or more processors can be further configured to: determine, according to the anti-counterfeiting code, whether the consumable is a counterfeit; display a message indicating that the consumable is a counterfeit and terminate the detection when the consumable is a counterfeit; and perform a further detection when the consumable is not a counterfeit.

In some embodiments, the test information can comprise a category of detection, a consumable number and a consumable batch number. The one or more processors can be further configured to: determine, according to the category of detection, the consumable number and the consumable batch number, whether the consumable is a product being recalled; display a message indicating that the consumable is a product being recalled and terminate the detection when the consumable is a product being recalled; and perform a further detection when the consumable is not a product being recalled.

In some embodiments, the test information can comprise date of production and expiration date. The one or more processors can be further configured to: determine, according to the date of production, the expiration date and a current time of the detection instrument, whether the consumable is expired; display a message indicating that the consumable is expired and terminate the detection when the consumable is expired; and perform a further detection when the consumable is not expired.

In some embodiments, the test information can comprise a limit for consumable reaction time. The one or more processors can be further configured to: determine, by comparing an actual reaction time of the consumable and the limit for consumable reaction time, whether a consumable reaction time is beyond limit; display a message indicating that the consumable reaction is beyond limit and terminate the detection when the consumable reaction time is beyond limit; and perform a further detection when the consumable reaction time is not beyond limit.

In some embodiments, the detection instrument can be configured to, once the consumable cassette is inserted into the detection instrument, write into the circuit board of the consumable cassette a current system time as an actual reaction start time of the consumable. The actual reaction start time of the consumable can be not deleted or modified. The actual reaction time of the consumable can be determined from the actual reaction start time of the consumable and the current system time.

In some embodiments, the test information can comprise a limit of number of read-write times. The one or more processors can be further configured to: determine whether the consumable is overused by comparing an actual number of read-write of the consumable cassette with the limit for number of read-write, said actual number of read-write of the consumable cassette being stored in the circuit board of the consumable cassette; display a message indicating that the consumable is overused when the consumable is overused; and perform a further detection when the consumable is not overused.

In some embodiments, the actual number of read-write of the consumable cassette can be increased by one each time the consumable cassette is inserted into the detection instrument. The actual number of read-write times of the consumable cassette can be not deleted or modified to a smaller number. The one or more processors can be further configured to perform the further detection when the actual number of read-write times of the consumable cassette is zero.

In some embodiments, the test information can comprise a threshold for insufficient sampling volume. The one or more processors are further configured to: determine whether the sampling volume of the consumable is insufficient by comparing a calculated value of a pre-scanned data of the consumable by the detection instrument with the threshold for insufficient sampling volume; display a message indicating that the sampling volume of the consumable is insufficient and terminate the detection when the sampling volume of the consumable is insufficient; and perform a further detection when the sampling volume of the consumable is sufficient.

In some embodiments, the calculated value of the pre-scanned data can be an intensity of a signal. In some instances, the one or more processors can be further configured to direct the detection instrument to perform a sampling at one or more positions of a detection side of the consumable at multiple time points, and average multiple sampling values to obtain the calculated value of the pre-scanned data. In some instances, the one or more processors can be configured to: direct the detection instrument to perform sampling at multiple positions of the detection side of the consumable at multiple time points, and average multiple sampling values to obtain the calculated value of the pre-scanned data.

In some embodiments, the test information can comprise a threshold for reaction over time failure. The one or more processors can be further configured to: determine whether the consumable reaction time is unreasonably long by comparing a calculated value of a pre-scanned data of the consumable by the detection instrument with the threshold for reaction over time failure; display a message indicating that the consumable reaction time is unreasonably long and terminate the detection when the consumable reaction time is unreasonably long; and perform a further detection when the consumable reaction time is not unreasonably long.

In some embodiments, the calculated value of the pre-scanned data can be an intensity of a signal. In some instances, the one or more processors can be further configured to direct the detection instrument to perform a sampling at one or more positions of a detection side of the consumable and/or at one or multiple time points, and average multiple sampling values to obtain the calculated value of the pre-scanned data. In some instances, the one or more processors can be further configured to: direct the detection instrument to perform sampling at multiple positions of the detection side of the consumable and/or at multiple time points, and average multiple sampling values to obtain the calculated value of the pre-scanned data.

In some embodiments, the test information can comprise a threshold for consumable failure. The one or more processors can be further configured to: determine whether the consumable fails by comparing a calculated value of a control line data scanned by the detection instrument with the threshold for consumable failure; display a message indicating that the consumable fails and terminating the detection when the consumable fails, and calculate and display a measurement result when the consumable does not fail.

In some embodiments, the calculated value of the control line data can be a peak height and/or peak area of a control line.

In some embodiments, the peak height and/or peak area of the control line can be calculated from a signal intensity of control line measured by the detection instrument in combination with a center point position, a half-peak width and a baseline width of consumable control line contained in the test information.

In some embodiments, the detection result can comprise a single signal or a set of signals.

In some embodiments, the one or more processors can be further configured to: calculate the detection result from a test line signal intensity of the consumable measured by the detection instrument in combination with a center point position a half-peak width and a baseline width of consumable test line contained in the test information.

In some embodiments, the one or more processors can be further configured to: calculate a measurement result and a gear level of measurement result of target object based on the detection result in combination with a calibration curve, a correction parameter and a critical value of result gear level contained in the test information.

In some embodiments, the one or more processors can be further configured to update the test information stored in the circuit board of the consumable cassette.

Various technical advantages are associated with the present disclosure over the prior art.

A circuit board configured to store test information is provided to the consumable cassette, such that information of the consumable can be read from the circuit board by the detection instrument. The circuit board does not increase a volume of the consumable cassette as it is embedded in the consumable cassette. No additional device (for example, a reflection light path acting as a part of a barcode scanning channel) is required in reading the test information as the detection instrument, to which a function of reading the test information is integrated, can read the test information upon an electrical connection with the circuit board. Therefore, a volume of the detection device can be reduced and a cost can be reduced.

### BRIEF DESCRIPTION OF THE DRAWINGS

**FIG. 1** is a side view of a consumable cassette for a detection device in accordance with an embodiment of the present disclosure;
**FIG. 2** is a top view of the consumable cassette of **FIG. 1**;
**FIG. 3** is a bottom view of the consumable cassette of **FIG. 1**;
**FIG. 4** is a block diagram of a detection instrument for a detection device in accordance with an embodiment of the present disclosure;
**FIG. 5** shows a use of the consumable cassette of **FIG. 1**;
**FIG. 6** is a flowchart of a detection method in accordance with an embodiment of the present disclosure;
**FIG. 7** is a flowchart of detecting whether a consumable cassette is correctly installed in accordance with an embodiment of the present disclosure;
**FIG. 8** is a flowchart of determining whether a consumable is in compliance with a detection requirement according to test information in accordance with an embodiment of the present disclosure;

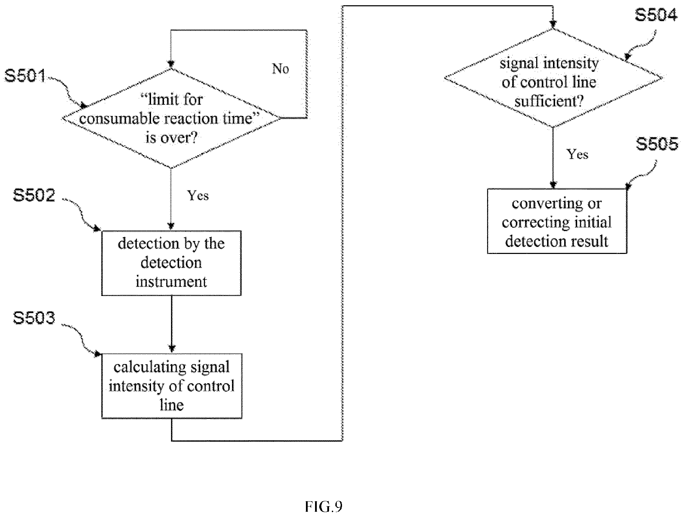

**FIG. 9** is a flowchart of detecting a consumable by a detection instrument in accordance with an embodiment of the present disclosure.

### DETAILED DESCRIPTION OF THE PRESENT DISCLOSURE

As described hereinabove, the cost in obtaining test information can be high with existing in vitro detection device.

The present disclosure provides a detection device and a detection method. A circuit board configured for storing test information is provided to the consumable cassette, such that test information can be read from the circuit board by the detection instrument. The circuit board does not increase a volume of the consumable cassette as it is embedded in the consumable cassette. No additional device is required in reading the test information as the detection instrument, to which a function of reading the test information is integrated, can read the test information upon an electrical connection with the circuit board. Therefore, a volume of the detection device can be reduced and a cost can be reduced.

As used herein, the term "consumable" generally comprises, but is not limited to, a test strip, a detection reagent, a reagent loading device and a biochip. The consumable can be disposable or reusable. The consumable can carry one or more reagent for detecting a presence and/or a content of one or more objects to be detected. The consumable can be used together with a detection instrument. Alternatively, the consumable can be used separately.

As used herein, the term "consumable cassette" generally refers to a device that encloses or carries a consumable. The consumable cassette can comprise a housing that at least partially encloses or carries the consumable therein. The consumable cassette can comprise an aperture or a window through which an object to be detected is applied to the consumable. The consumable cassette can be provided with a mechanical means and/or an electronic means for fixing the consumables and applying the objects to be detected.

As used herein, the term "detection instrument" generally refers to a device that reads a test result from the consumable or the consumable cassette. The detection instrument can be a mechanical device, an electronic device or a combination thereof. For example, the detection instrument can at least partially enclose the consumable or the consumable cassette, such that the test result can be read in an optical manner, in an electronical manner, in a magnetic manner, in a visual manner, or a combination thereof. Alternatively, the detection instrument can read a test information or test result from the consumable or the consumable cassette in a non-contact manner. The detection instrument can store and display a user setting, a test parameter and a test result. The detection instrument can be capable of communication. For example, the detection instrument can be connected with other devices via a network or a communication link.

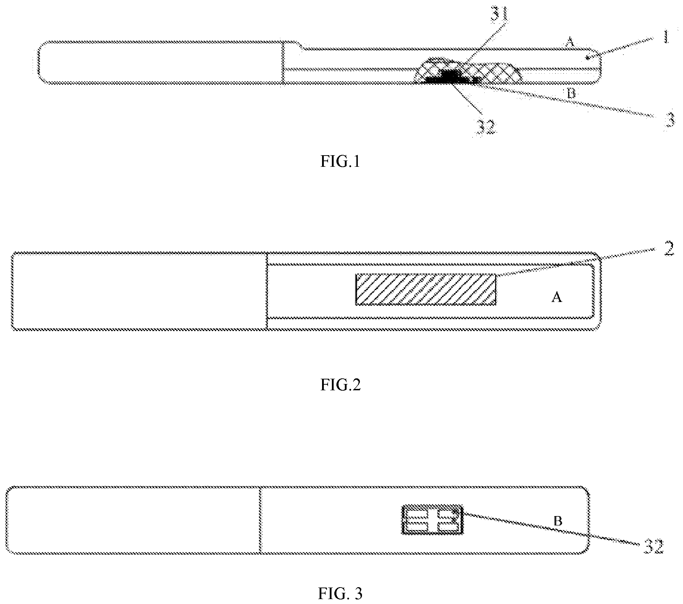

**FIG. 1** is a side view of a consumable cassette for a detection device in accordance with an embodiment of the present disclosure. The detection device can comprise a consumable cassette **1** and a detection instrument (not shown). The consumable cassette can comprise a detection side **A** on which a consumable is provided. The consumable cassette can be provided with a circuit board **3**. The circuit board can be provided at various position of the consumable cassette. For instance, the circuit board can be enclosed within the consumable cassette. For instance, the circuit board can be fixed at an end of the consumable cassette. For instance, the circuit board can be embedded into a surface of the consumable cassette. In some embodiments, the consumable cassette can comprise a back side **B** which is opposite to the detection side **A**. The circuit board **3** can be embedded into the back side **B**. Alternatively, the circuit board **3** can be embedded into the detection side **A**. Test information can be provided in the circuit board **3**. The detection instrument can be configured to read the test information from the circuit board **3** and perform a detection on the consumable based on the test information to obtain a detection result.

In some embodiments, the consumable in the consumable cassette **1** can be a test strip. The test strip can hold therein a blood or a urine to effect an in vitro detection. Alternatively, the consumable in the consumable cassette **1** can be other types of materials which are capable of carrying a liquid sample to be detected, a solid sample to be detected, or a mixture of solid and liquid samples to be detected. In some embodiments, a portion of the sample, which is applied to a first portion of the consumable, can be transferred to a second portion of the consumable the consumable by a capillary action. For instance, a liquid sample applied to a first end of the consumable can be transferred to a second end of the consumable by capillary action within 1 second, 2 seconds, 3 seconds, 4 seconds, 5 seconds, 10 seconds, 15 seconds, 20 seconds, 25 seconds, 30 seconds, 40 seconds, 50 seconds or 60 seconds. The first end can be a sample loading area, and the second end can be a sample detection area.

In some instances, the consumable cassette **1** can be provided with a plate configuration. **FIG. 2** is a top view of the consumable cassette of **FIG. 1. FIG. 2** shows a schematic view of the detection side **A**. A detection area **2** can be provided on the detection side **A** where a test strip is disposed. The detection device of the disclosure can be configured to perform an in vitro detection to a body fluid carried in the test strip within the detection area **2** and obtain a detection result.

In some instances, the circuit board **3** can be embedded into the back side **B** of the consumable cassette **1**. The test information can be provided in the circuit board **3**, which information can be read out in the process of detection. The circuit board can be accommodated within a rather small space as it is embedded in the consumable cassette. No additional device (e.g., a reflection light path) is required in reading the test information as the detection instrument can read the test information upon an electrical connection with the circuit board **3**. Therefore, a cost can be reduced. In some instances, the circuit board **3** can comprise a chip **31** embedded into the back side of the consumable cassette for storing the test information. The chip **31** can be protected from damage as it is embedded in the consumable cassette.

In some instances, the chip **31** can be a read/write microchip having a small size. The read/write microchip can be embedded in the consumable cassette **1**. The chip **31** can be a contact chip. The chip **31** can be a contactless chip, such as a radio frequency chip.

Various data can be stored in the chip **31**. Various information about the consumable can be obtained by the detection instrument in performing a detection process, such that a mistake in using the consumable can be avoided, and an accuracy and an efficiency of the detection can be improved. In some instances, the test information stored in the chip **31** can comprise, but not limited to, one or more of an anti-counterfeiting code, a category of detection, a consumable number, a consumable batch number, date of production, expiration date, time of first read/write, number of read/write operations, a limit for number of read-write, a limit for consumable reaction time, a threshold for insufficient sampling volume, name of test line, name of control line, a parameter of signal pre-processing (e.g., a center point position of test line, a center point position of control line, a half-peak width, and a baseline width), a threshold for reaction over time failure, a threshold for consumable failure, a calibration curve (e.g., type of curve, and parameters of curve), a correction parameter, a critical value of result gear level, and an unit of testing. The detection instrument can read the pre-stored information from the chip **31**. The detection instrument can write information into the chip **31**. For instance, the detection instrument can be configured to write a test result into the chip **31** or modify the information pre-stored in the chip **31**.

**FIG. 3** is a bottom view of the consumable cassette of **FIG. 1. FIG. 3** shows a schematic view of a structure of a back side **B**. The circuit board **3** can further comprise a first connection end **32** at the back side for electrically connecting the chip to the detection instrument. The first connection end **32** can be provided as a connection structure that matches a connection end of the detection instrument.

In some embodiments, the first connection end **32** can comprise a contact plate covering the back side B of the consumable cassette 1. The contact plate can be connected to the chip **31**, such that the chip **31** is connected to an external circuit (for example, a reading unit of the detection instrument). The contact plate can be provided in a matrix. For instance, as shown in **FIG. 3**, the contact plates can comprise four thin metal plates which are disposed on the back side of the consumable cassette in a 2 x 2 matrix. In some embodiments, the circuit board **3** can be disposed at a position corresponding to the detection area **2**. For instance, a projection of the circuit board **3** on the detection side **A** can be within a preset area of the detection area **2**, such that the circuit board **3** can be used to determine whether the detection area **2** is within a detectable range during the detection.

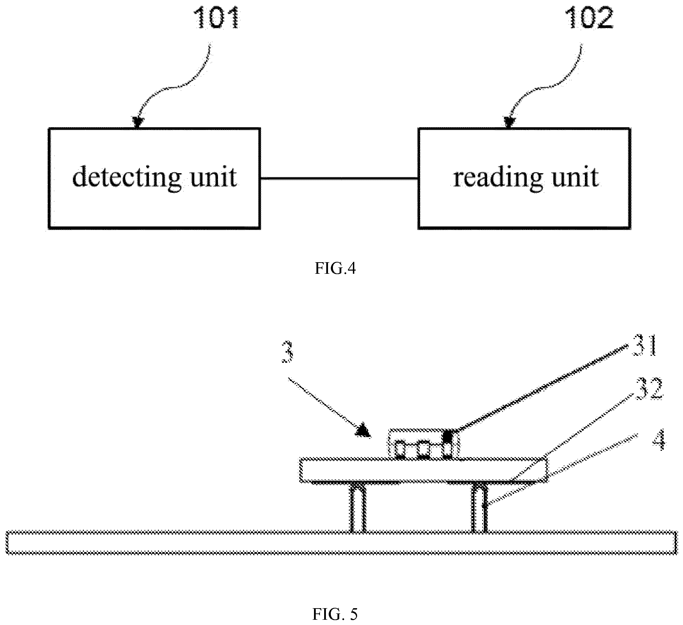

In some embodiments, the detection device can further comprise a detection instrument. **FIG. 4** is a block diagram of a detection instrument in accordance with an embodiment of the present disclosure.

The detection instrument can comprise a detecting unit **101** configured to detect a test strip in the consumable cassette. For instance, the detecting unit **101** can be provided as an optical and mechanical system which is configured to scan and measure an optical response signal from the test strip. In some embodiments, the optical and mechanical system can be movable relative to the consumable cassette, such that detection signals at multiple locations in the detection area of the consumable can be read. In some embodiments, the detection instrument can be further configured to read the test information from the circuit board **3**. For instance, the detection instrument can comprise a reading unit **102** which is electrically connected to the circuit board **3** when the consumable cassette is placed in the detection instrument, such that the test information can be read from the circuit board **3**. The reading unit **102** can be, for example, a contact chip reading device or a contactless chip reading device. In some instances, the chip **31** of the circuit board can be a contactless chip, and the reading unit **102** can be a contactless chip reading device. The detecting unit **101** can comprise a data processing instrument such as a computer to obtain a detection report. The reading unit **102** can be integrated into the computer to simplify a configuration of the detection device.

**FIG. 5** shows a use of the consumable cassette of **FIG. 1**. In some embodiments, the detection instrument can comprise a slot (not shown in the figure) configured to receive the consumable cassette. A second connection end **4**, which is connected to the reading unit, can be provided in the slot. The first connection end **32** at the back side of the consumable cassette can be connected to the second connection end **4** of the detection instrument when the consumable cassette is inserted into the slot of the detection instrument, such that the reading unit of the detection instrument is electrically connected to the circuit board **3** of the consumable cassette.

In some embodiments, the second connection end **4** of the detection instrument can comprise contact points that match the first connection end **32** (for example, contact plates) of the consumable cassette. The second connection end **4** can be in contact with the first connection end **32** when the consumable cassette is placed in position in the slot. In some embodiments, the contact plates of the consumable cassette can comprise four thin metal plates which are disposed at the back side of the consumable cassette in a 2×2 matrix. Accordingly, the contact points of the detection instrument can comprise four metal projections which are disposed at a bottom of the slot in a 2×2 matrix. The metal projections can abut against the thin metal plates when the consumable cassette is inserted into the slot, such that the contact points are electrically connected to the contact plates, and an electrical connection between the reading unit **102** and the circuit board **3** is effected.

Once the reading unit **102** of the detection instrument is electrically connected to the circuit board 3 of the consumable cassette, the reading unit **102** in the detection instrument can read the test information from the circuit board **3** and detect the consumable based on the test information to obtain a detection result.

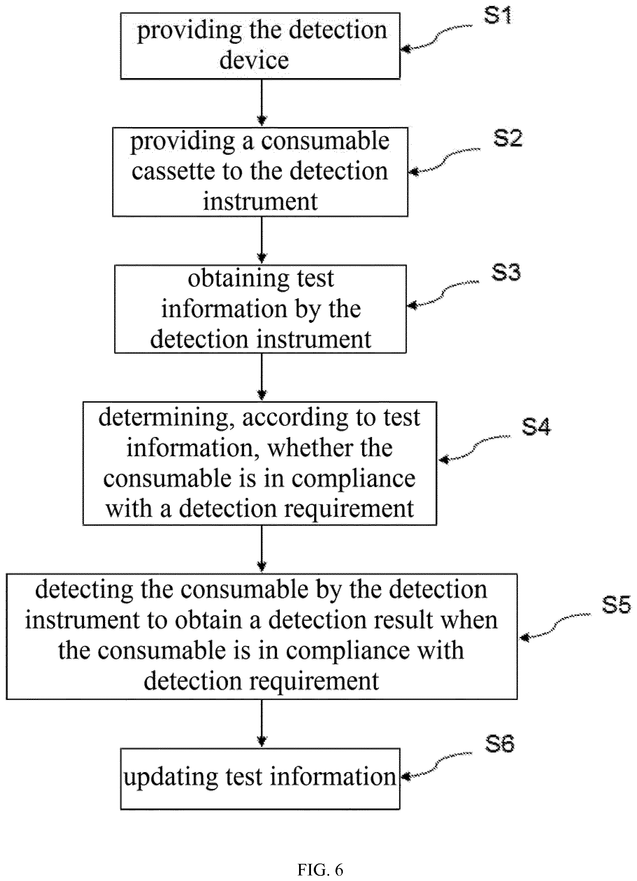

The present disclosure also provides a detection method with the detection device of the present disclosure. **FIG. 6** is a flowchart of a detection method in accordance with an embodiment of the present disclosure. In some embodiments, the detection method can comprise processes **S1** to **S6**.

In process **S1**, the detection device can be provided.

In process **S2**, the consumable cassette can be provided to the detection instrument;

In process **S3**, the test information can be obtained by the detection instrument;

In process **S4**, a determination can be made, according to the test information, on whether the consumable is in compliance with a detection requirement.

In process **S5**, the consumable can be detected by the detection instrument to obtain a detection result when the consumable is in compliance with the detection requirement; and

In process **S6**, the test information can be updated once the detection result is obtained.

The processes of the detection method will be described in detail with reference to the schematic views illustrating a configuration of the detection device as shown in **FIGs. 1-5**.

In process **S1**, the detection device can be provided. A configuration of the detection device can be substantially the same as the configuration of the detection device described hereinabove.

In process **S2**, the consumable cassette **1** can be provided to the detection instrument. In some embodiments, the consumable cassette **1** can comprise a detection side **A** on which a consumable is provided and a back side **B** which is opposite to the detection side **A**. The circuit board **3** can be embedded into the back side **B**. Test information can be provided in the circuit board **3**. The detection instrument can comprise a slot. The detection instrument can comprise a detecting unit **101** and a reading unit **102**. The detecting unit **101** can be configured to detect a test strip in the consumable cassette.

The process of providing the consumable cassette **1** to the detection instrument can comprise placing the consumable cassette **1** into the slot in a manner where the back side **B** of the consumable cassette **1** faces a bottom of the slot and the detection side **A** of the consumable cassette **1** faces the detecting unit **101**.

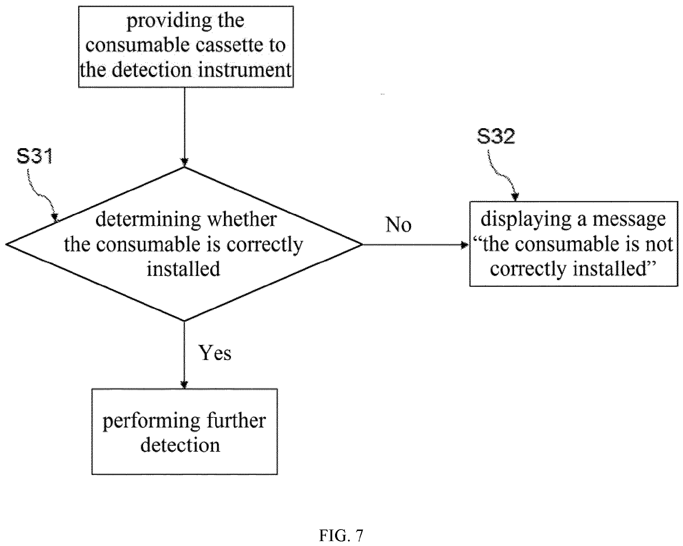

In process **S3**, the test information can be obtained by the detection instrument. **FIG. 7** is a flowchart of process **S3** in accordance with an embodiment of the present disclosure. The process S3 can comprise processes **S31** to **S32**. In process **S31**, a determination can be made on whether the consumable is correctly installed. In some embodiments, a correct installation of the consumable can be determined by detecting whether the second connection end **4** of the detection instrument is in a connected state, such that a determination on whether the detection area of the consumable is at a detectable position of the detecting unit of the detection instrument can be made. The process **S4** can be performed if it is determined that the consumable cassette is correctly installed, in which process **S4** a determination can be made, according to the test information, on whether the consumable is in compliance with a detection requirement. Otherwise, if it is determined that the consumable cassette is not correctly installed, then a process **S32** can be performed to display a message "the consumable is not correctly installed" on an interface of the detection instrument.

For instance, the detecting unit of the detection instrument can correctly detect the detection area **2** of the consumable when the consumable cassette is correctly installed in the slot of the detection instrument. In this state, the first connection end **32** opposite to the detection area **2** can be electrically connected to the second connection end 4 which is provided at the bottom of the slot of the detection instrument. Therefore, a determination on whether the consumable cassette is correctly installed can be determined by detecting whether the second connection end **4** of the detection instrument is in a connected state. On the contrary, a position of the first connection end **32** of the consumable can fail to correspond to a position of the second connection end **4** if the consumable cassette is not correctly installed in the slot, resulting in no electrical connection being established between the first connection end **32** and the second connection end **4** and the second connection end **4** being in a disconnected state. A determination can be made that the consumable cassette is not correctly installed. In this state, the process **S32** can be performed to display a message "the consumable cassette is not correctly installed" on the interface of the detection instrument, and the detection process can be terminated.

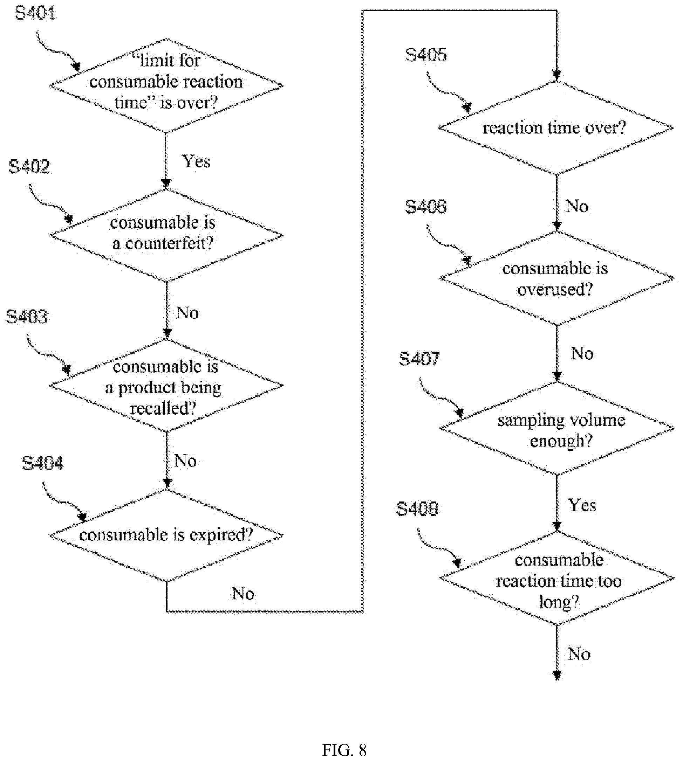

**FIG. 8** is a flowchart of determining whether a consumable is in compliance with a detection requirement according to test information in accordance with an embodiment of the present disclosure. The process **S4** can be performed to determine, according to the test information, on whether the consumable is in compliance with a detection requirement when the consumable is correctly installed. In some embodiments, the test information can comprise, but not limited to, one or more of an anti-counterfeiting code, a category of detection, a consumable number, a consumable batch number, date of production, expiration date, time of first read/write, number of read/write operations, a limit for number of read-write, a limit for consumable reaction time, a threshold for insufficient sampling volume, name of test line, name of control line, a parameter of signal pre-processing (e.g., center point position of test line, center point position of control line, a half-peak width, and a baseline width), a threshold for reaction over time failure, a threshold for consumable failure, a calibration curve (e.g., type of curve, and parameter of curve), a correction parameter, a critical value of result gear level, and an unit of testing.

In some embodiments, the process **S4** can comprise determining whether a parameter of the consumable is in compliance with the detection requirement according to the specific content of the test information, which test information is read from the consumable. The process **S4** can comprise processes **S401** to **S408**. In process **S401**, a determination can be made on whether the detection instrument can correctly read data from the circuit board of the consumable. The determination can be effected by performing a data reading and writing test on the chip of the consumable. For instance, a preset data can be written to the chip of the consumable, and the preset data can then be read from the chip of the consumable. The determination can be made on whether the detection instrument can correctly read data from the circuit board of the consumable by comparing a consistency between the data which is written to the chip of the consumable and the data which is read from the chip of the consumable, a data write speed, a data read speed and other related information.

An message indicating the consumable being not readable can be displayed on the interface of the detection instrument and the detection process can be terminated if it is determined in process **S401** that the detection instrument is not able to correctly read data from the circuit board of the consumable and/or correctly write data to the circuit board of the consumable. A further detection process can be performed according to the test information only if a determination is made in process **S401** that the detection instrument can correctly read data from the circuit board of the consumable. In this way, a consumable having a faulty circuit board can be recognized by the detection instrument, such that an invalid measurement can be avoided.

In some instances, the test information can comprise an anti-counterfeiting code. In this case, the process of determining, according to the test information, whether the consumable is in compliance with the detection requirement can comprise a process **S402** for determining, according to the anti-counterfeiting code, whether the consumable is a counterfeit. The anti-counterfeiting code can be set of data which is unique to each consumable, which data can be a combination of a number, a letter and a character. In some instances, the detection device can determine whether the corresponding consumable is a counterfeit by determining whether the anti-counterfeiting code conforms to a preset format. In some instances, the detection device can store therein an anti-counterfeiting code verification algorithm to verify the anti-counterfeiting code locally. In some instances, the detection device can be configured to communicate with a remote server over a wireless communication link once the anti-counterfeiting code is read, send the anti-counterfeiting code to the server for verification, and receive a verification result from the server. The wireless communication link can be, for example, a cellular communication network, Wi-Fi, or Bluetooth.

A message indicating the consumable being a counterfeit can be displayed on the interface of the detection instrument and the detection process can be terminated if it is determined in process **S402** that the consumable is a counterfeit. A further detection process can be performed only if it is determined that the consumable is not a counterfeit. In this way, a counterfeit consumable can be rejected to avoid an inaccurate detection result.

In some instances, the test information can comprise a category of detection, a consumable number and a consumable batch number. In this case, the process of determining, according to the test information, whether the consumable is in compliance with the detection requirement can comprise a process **S403** for determining, according to a category of detection, a consumable number and a consumable batch number, whether the consumable is a product being recalled. The detection instrument can be configured to communicate with a remote server in a wired or wireless manner to obtain an updated product information, including a product update information, a product defect information and a product recall index. According to the category of detection, the consumable number and the consumable batch number in the test information, the detection instrument can determine the attribute information of the corresponding consumable, including a category of detection, a manufacturer, a production batch, a defective product being recalled or not.

A message indicating the consumable being a recalled product can be displayed on the interface of the detection instrument and the detection process can be terminated if it is determined in process **S403** that the consumable is a product being recalled. A further detection process can be performed only if it is determined that the consumable is not a product being recalled. In this way, a probability of using a consumable which is not in compliance with detection requirement can be reduced, and a detection accuracy can be improved.

In some instances, the test information can comprise date of production and expiration date. In this case, the process of determining, according to the test information, whether the consumable is in compliance with the detection requirement can comprise process a **S404** for determining, according to the date of production and the expiration date, whether the consumable is expired. A message indicating the consumable being expired can be displayed on the interface of the detection instrument and the detection process can be terminated if it is determined in process **S404** that the consumable is expired. A further detection process can be performed only if it is determined that the consumable is not expired. In this way, a use of expired consumable can be prevented, and an efficiency and accuracy of the detection can be improved.

In some instances, the test information can comprise a limit for consumable reaction time. In this case, the process of determining, according to the test information, whether the consumable is in compliance with the detection requirement can comprise a process **S405** for determining, by comparing an actual reaction time of the consumable and the limit for consumable reaction time, whether the reaction time of the consumable is beyond the limit. Once a target sample (for example, blood, or urine) is applied to the detection side of the consumable, the user is required to insert the consumable cassette into the detection instrument in a timely manner. For example, the user is required to insert the consumable cassette into the detection instrument within 30 seconds according to a standard operation procedure. In detecting a sample, a sufficient reaction time can be required to ensure a complete reaction between the sample and a reactant carried on the consumable to obtain an accurate detection result. On the other hand, an unacceptable long reaction time can result in an inaccurate detection result. For instance, a fluorescence signal can be increased abnormally under an unacceptable long reaction time. Therefore, an accurate detection result can be expected if the reaction time between the sample and the reactant carried on the consumable is within a reasonable range and the detection result is read within this range. The limit for consumable reaction time can vary according to a category of detection. In some instances, the limit for consumable reaction time can be 10 to 20 minutes.

In some instances, once the consumable cassette is inserted into the detection instrument, the detection instrument can write into the chip **31** of the consumable cassette a current system time as an actual reaction start time of the consumable. Once written, the actual reaction start time of the consumable cannot be deleted or modified. The actual reaction time of the consumable can be determined from the actual reaction start time of the consumable recorded in the chip **31** and current system time. Even if the user removes the consumable cassette from the detection instrument after the initial inserting and inserts the consumable cassette into the detection instrument again or into another detection instrument, the actual reaction time of the consumable can still be determined as the actual reaction start time of the consumable recorded in the chip **31** of the consumable cassette cannot be modified.

A message indicating the actual reaction time of the consumable exceeding the limit for consumable reaction time can be displayed on the interface of the detection instrument and the detection process can be terminated if it is determined in process **S405** that the actual reaction time of the consumable exceeds the limit for consumable reaction time. A further detection process can be performed only if the actual reaction time of the consumable does not exceed the limit for consumable reaction time. In this way, an unacceptable reaction time, which can be caused by repeated insertion and removal of the consumable, can be prevented, and an efficiency and an accuracy of the detection can be improved.

In some instances, the test information can comprise a limit for number of read-write. In this case, the process of determining, according to the test information, whether the consumable is in compliance with the detection requirement can comprise a process **S406** for determining whether the consumable is overused by comparing an actual number of read-write of the consumable with the limit for number of read-write. For instance, upon an initial insertion of the consumable cassette into the detection instrument, the detection instrument can update "number of read-write", which is stored in the chip **31** of the consumable cassette, to "one". The detection instrument can update "number of read-write", which is stored in the chip **31** of the consumable cassette, to "two" if the user removes the consumable cassette from the detection instrument and inserts the consumable cassette again. Once written, the number of read-write cannot be deleted or modified to a smaller value. In some instances, the limit for number of read-write can be five.

A message indicating the consumable being overused can be displayed on the interface of the detection instrument and the detection process can be terminated if it is determined in process **S406** that the number of read-write of the consumable exceeds the limit for number of read-write. A further detection process can be performed only if it is determined that the number of read-write of the consumable does not exceed the limit for number of read-write. In this way, a deactivation of the consumable or an unacceptable long reaction time caused by inserting and removing the consumable multiple times can be prevented, thereby improving an efficiency and an accuracy of the detection process.

In some instances, the process **S405** can be bypassed if the detection instrument determines that "number of read-write" shored in the chip **31** of the consumable cassette is zero (that is, the consumable cassette is used for the first time) . In this case, the number of read-write can be set to "one" and a further detection process can be performed.

In some instances, the test information can comprise a threshold for insufficient sampling volume. In this case, the process of determining, according to the test information, whether the consumable is in compliance with the detection requirement can comprise a process **S407** for determining whether the sampling volume of the consumable is sufficient by comparing a calculated value of a pre-scanned data of the consumable with the threshold for insufficient sampling volume. In some embodiments, a preliminary determination on whether the sampling volume is sufficient can be made prior to an actual reaction and detection. For instance, a signal can be generated at the detection area of the consumable upon an application of sample to be detected (for example, blood or urine). The signal can be a fluorescent signal or a non-fluorescent signal. Examples of non-fluorescent signals can comprise a light absorption signal, an electrical signal, a magnetic signal or a color signal. An intensity of the signal can indicate an amount of the sample which is applied to the detection area of the consumable. The detection instrument can be configured to detect the intensity of the signal at one or more positions. If the intensity of the signal is greater than a threshold, then a determination can be made that the sampling volume of the sample at the position is large enough to perform an accurate detection. In some instances, the detection instrument can be configured to perform a sample volume sampling at one sampling point of the consumable only once to obtain a sampling value. Alternatively, the detection instrument can be configured to perform a sample volume sampling on one sampling point of the consumable at multiple time points and average the multiple sampling values to obtain an average value. If the sampling value or average value is less than the threshold for insufficient sampling volume, then a determination can be made that the sampling volume of the consumable is too small to perform an accurate detection. In some instances, the detection instrument can be configured to perform a sample volume sampling at a plurality of different sampling points on the consumable (for example, at 200 different sampling points) and perform calibration in multiple gear level during the scanning process. Subsequently, sampling values at 50 to 100 sampling points from among the 200 sampling points can be selected and assembled into an array. The array can be averaged to obtain an average value. The sampling at the plurality of sampling points can be performed at different time points. Alternatively, the sampling at the plurality of sampling points can be performed simultaneously. If the average value is less than the threshold for insufficient sampling volume, then a determination can be made that the sampling volume of the consumable is too small to perform an accurate detection. The sample sampling at multiple sampling points can be effected by a movement of the consumable, a movement of the detecting unit of the detection instrument, or a movement of both the consumable and the detecting unit.

A message indicating the sampling volume of the consumable is not sufficient for detection can be displayed on the interface of the detection instrument and the detection process can be terminated if it is determined in process **S407** that the sampling volume of the consumable is too small to perform a detection. A further detection process can be performed only if it is determined that the sampling volume exceeds the threshold for insufficient sampling volume (e.g., the sampling volume of the consumable is in compliance with a detection requirement). In this way, a low signal intensity resulting from the insufficient sampling volume can be avoided, and an accuracy of the detection can be improved.

In some instances, the test information can comprise a threshold for reaction over time failure. In this case, the process of determining, according to the test information, whether the consumable is in compliance with the detection requirement can comprise a process **S408** for determining whether the consumable reaction time is unreasonably long by comparing a calculated value of a pre-scanned data of the consumable with the threshold for reaction over time failure. As described hereinabove in the disclosure, a fluorescent signal can be generated at the detection area of the consumable upon an application of sample to be detected (for example, blood or urine). An intensity of the fluorescent signal can be substantially constant within a time window (e.g., within a limit of consumable reaction time). Beyond the time window, however, the intensity of the fluorescent signal can be increase due to an accumulation of the sample. Therefore, a determination on whether the current time is within the time window suitable for detection (for example, whether the current time is within the limit of consumable reaction time) can be made by comparing the intensity of the detected fluorescent signal with the threshold for reaction over time failure. If the intensity of the detected fluorescent signal exceeds the threshold for reaction over time failure, then a determination can be made that the sample is applied to the consumable a while ago and thus is not suitable for detection. In some instances, the detection instrument can be configured to perform a sample volume sampling at 200 different sampling points on the consumable and perform calibration in multiple gear level during the scanning process. Subsequently, sampling values at 50 to 100 sampling points from among the 200 sampling points can be selected and assembled into an array. The array can be averaged to obtain an average value of the fluorescent signals. The average value of the fluorescent signals can be compared to the threshold for reaction over time failure to determine whether the current time is within the time window suitable for detection.

A message indicating the reaction time is too long to perform a detection can be displayed on the interface of the detection instrument and the detection process can be terminated if it is determined in process **S408** that the consumable reaction time is too long and thus is not suitable for sample detection. A further detection process can be performed only if it is determined that the consumable reaction time is suitable for sample detection. In this way, a signal error resulting from inserting a consumable into the detection instrument long after a sample is applied to the consumable, such that an accuracy of the detection can be improved. For instance, a situation where the user does not insert the consumable cassette, to which the sample is applied, into the detection instrument in a timely manner can be avoided.

Those skilled in the art will appreciate that, processes of performing various detections on the consumable based on content of the test information can be executed individually or collectively, and a sequence of executing the processes is not limited to the exemplary embodiments described hereinabove.

The process **S5** for detecting the consumable by the detection instrument can be performed if a determination is made in process **S4** that the consumable is in compliance with a detection requirement. In some instances, the detection instrument can be configured to obtain one single scan signal or a set of scan signals. In some instances, the instrument can be a one-dimensional scanning detection instrument. In this case, a set of scan signals such as a set of scan signals in a format [Scan Position, Signal Intensity] can be obtained as a result of the process **S5**.

In some embodiments, one or more effective signal intensities of test line and/or one or more effective signal intensities of control line can be calculated based on the parameter of signal pre-processing which is stored in chip **31** of the circuit board **3**. In some instances, a process **S503** can be performed where an effective signal intensity of the control line (e.g., a peak height and/or a peak area of the control line) can be calculated from the set of scan signals in combination with the parameter of signal pre-processing (e.g., a center point position of control line, a half-peak width, and a baseline width of the consumable). In some instances, an effective signal intensity of the test line (e.g., a peak height and/or a peak area of the test line) can be calculated from the set of scan signals in combination with the parameter of signal pre-processing (e.g., a center point position of test line, a half-peak width, and a baseline width of the consumable). It will be appreciated that the consumable may not comprise a control line.

In some embodiments, a determination can be made on whether a consumable fails based on the effective signal intensity of the control line. In some instances, a process **S504** can be performed where a determination can be made on whether the consumable fails based on the effective signal intensity of the control line and the threshold for consumable failure contained in the test information. For instance, a determination on whether the consumable fails can be made by confirming whether the effective signal intensity of the control line is within an acceptable range. A message indicating the consumable fails can be displayed on the interface of the detection instrument and the detection process can be terminated if it is determined in process **S504** that the effective signal intensity of control line is not within the acceptable range. A further detection process can be performed only if it is determined that the consumable is in a normal state. In this way, a consumable having a failed control line due to external interference can be identified, and thus an accuracy of the detection can be improved.

In some embodiments, a detection result of the detected object/tested object, such as a concentration, negative/positive, and a gear level of detection result (such as high, medium, and low), can be calculated or obtained based on the one or more effective signal intensities of test line and the one or more effective signal intensities of control line in combination with a calibration curve, a correction parameter, and a critical value of result gear level contained in the test information. For instance, the test information, which is stored in the chip **31** of the consumable cassette, can comprise one or more of a calibration curve type, a curve parameter, a correction parameter, a critical value of result gear level and a measurement unit. In some instances, a process **S505** can be performed where an initial detection result (for example, the effective signal intensity of the fluorescent signal) is obtained can be converted or corrected based on the calibration curve type, the curve parameter, the correction parameter and the measurement unit read from the chip **31** of the consumable cassette, such that a more accurate detection result can be obtained. For instance, a qualitative analysis (e.g., negative/positive, high/medium/low) can be performed on the initial detection result based on the critical value of result gear level. It will be appreciated that the detection result can be directly output as the final detection result in case no conversion or correction is required.

In some embodiments, the detection result of the detected object/tested object can be associated with a name of the detected object/tested object based on the detection result of the detected object and the name of the consumable which is stored in the circuit board of the consumable,.

As shown in **FIG. 6**, in some instances, a process **S6** can be performed once the detection is completed to update the test information stored in the circuit board of the consumable cassette. For instance, the number of read-write in the test information can be increased by one when the detection is completed. In this way, the detection instrument can determine whether the consumable is reused by reading the number of read-write. In some instances, one or more of a date of detection, a time, a name of the object to be detected or measured, detection result of the detected object/tested object can be written into the chip of the circuit board of the consumable cassette once the detection is completed. The detection result can be read out, which enables the user to obtain the detection result. In addition, the written detection result can be used as a criterion in determining whether the consumable cassette is reused. For instance, if a previously written detection result is found while the test information is read, then a determination can be made that the consumable is reused.

With the detection method of the disclosure, the test information can be directly obtained from the consumable cassette or the detection instrument without any additional device, such that an accuracy of the detection result and an efficiency of the detection can be improved.

The foregoing disclosure is merely illustrative of preferred embodiments of the invention, and the protection scope of the invention is not limited thereto. Any equivalent modifications or variations made by those of ordinary skill in the art according to the disclosure of the invention shall fall into the protection scope of the disclosure.

## Claims

1. A detection device, comprising:
a consumable cassette configured to have a detection side provided with a consumable, wherein the consumable cassette is provided with a circuit board carrying a test information; and
a detection instrument configured to read the test information from the circuit board and perform a detection on the consumable based on the test information to obtain a detection result.

2. The detection device of claim 1, wherein the circuit board comprises a chip disposed on the circuit board for storing the test information.

3. The detection device of claim 1, wherein the circuit board comprises a first connection end which is electrically connected with the chip.

4. The detection device of claim 2, wherein the detection instrument comprises: a detecting unit configured to perform a detection on the consumable; and a reading unit configured to read the test information.

5. The detection device of claim 4, wherein the detection instrument further comprises a slot configured to receive the consumable cassette, and wherein a second connection end connected with the reading unit is provided in the slot.

6. The detection device of claim 5, wherein the first connection end comprises a contact plate covering an outer surface of the detection instrument, and wherein the second connection end comprises a contact point matching the contact plate.

7. The detection device of claim 5, wherein the first connection end comprises contact plates which are provided in a matrix, and wherein the second connection end comprises contact points which are provided in a matrix.

8. The detection device of claim 1, wherein the detection side is provided with a detection area through which the consumable is exposed.

9. The detection device of claim 1, wherein the test information carried in the chip comprises one or more of an anti-counterfeiting code, a category of detection, a consumable number, a consumable batch number, date of production, expiration date, time of first read/write, number of read/write operations, a limit for number of read-write, a limit for consumable reaction time, a threshold for insufficient sampling volume, name of test line, name of control line, a parameter of signal pre-processing, a threshold for reaction over time failure, a threshold for consumable failure, a calibration curve, a correction parameter, a critical value of result gear level, and an unit of testing.

10. The detection device of claim 9, wherein the parameter of signal pre-processing comprises one or more of a center point position of test line, a center point position of control line, a half-peak width and a baseline width.

11. The detection device of claim 9, wherein the calibration curve comprises a type of curve and/or a parameter of curve.

12. The detection device of claim 9, wherein the chip is a read/write chip, and wherein the chip is configured to further store a test result.

13. A detection method, comprising:
providing the detection device of any one of claims 1-12;
placing the consumable cassette into the detection instrument;
obtaining the test information by the detection instrument;
determining, according to the test information, whether the consumable is in compliance with a detection requirement; and
performing a detection on the consumable by the detection instrument to obtain a detection result when the consumable is in compliance with the detection requirement.

14. The detection method of claim 13, wherein obtaining the test information by the detection instrument comprises determining whether the consumable cassette is correctly installed; and
wherein determining, according to the test information, whether the consumable is in compliance with the detection requirement comprises reading the test information from the circuit board when the consumable cassette is correctly installed, and determining whether the consumable is in compliance with the detection requirement according to the test information.

15. The detection method of claim 13, wherein determining, according to the test information, whether the consumable is in compliance with the detection requirement comprises:
determining the consumable is readable when the detection instrument is able to read the test information from the circuit board and write a data into the circuit board;
displaying a message indicating that the consumable is not readable and terminating the detection when the consumable is not readable; and
performing a further detection when the consumable is readable.

16. The detection method of claim 13, wherein the test information comprises an anti-counterfeiting code, and
wherein determining, according to the test information, whether the consumable is in compliance with the detection requirement comprises:
determining, according to the anti-counterfeiting code, whether the consumable is a counterfeit;
displaying a message indicating that the consumable is a counterfeit and terminating the detection when the consumable is a counterfeit; and
performing a further detection when the consumable is not a counterfeit.

17. The detection method of claim 13, wherein the test information comprises a category of detection, a consumable number and a consumable batch number, and
wherein determining, according to the test information, whether the consumable is in compliance with the detection requirement comprises:
determining, according to the category of detection, the consumable number and the consumable batch number, whether the consumable is a product being recalled;
displaying a message indicating that the consumable is a product being recalled and terminating the detection when the consumable is a product being recalled; and
performing a further detection when the consumable is not to be a product being recalled.

18. The detection method of claim 13, wherein the test information comprises date of production and expiration date, and
wherein determining, according to the test information, whether the consumable is in compliance with the detection requirement comprises:
determining, according to the date of production, the expiration date and a current time of the detection instrument, whether the consumable is expired;
displaying a message indicating that the consumable is expired and terminating the detection when the consumable is expired; and
performing a further detection when the consumable is not expired.

19. The detection method of claim 13, wherein the test information comprises a limit for consumable reaction time, and
wherein determining, according to the test information, whether the consumable is in compliance with the detection requirement comprises:
determining, by comparing an actual reaction time of the consumable and the limit for consumable reaction time, whether a reaction time of the consumable is beyond limit;
displaying a message indicating that the reaction time of the consumable is beyond limit and terminating the detection when the consumable reaction time is beyond limit; and
performing a further detection when the consumable reaction time is not beyond limit.

20. The detection method of claim 19, wherein the detection instrument is configured to, once the consumable cassette is inserted into the detection instrument, write into the circuit board of the consumable cassette a current system time as an actual reaction start time of the consumable.

21. The detection method of claim 20, wherein the actual reaction start time of the consumable is not deleted or modified.

22. The detection method of claim 20, wherein the actual reaction time of the consumable is determined from the actual reaction start time of the consumable and the current system time.

23. The detection method of claim 13, wherein the test information comprises a limit for number of read-write, and
wherein determining, according to the test information, whether the consumable is in compliance with the detection requirement comprises:
determining whether the consumable is overused by comparing an actual number of read-write of the consumable cassette with the limit for number of read-write, said actual number of read-write of the consumable cassette being stored in the circuit board of the consumable cassette;
displaying a message indicating that the consumable is overused when the consumable is overused; and
performing a further detection when the consumable is not overused.

24. The detection method of claim 23, wherein the actual number of read-write of the consumable cassette is increased by one each time the consumable cassette is inserted into the detection instrument.

25. The detection method of claim 23, wherein the actual number of read-write of the consumable cassette is not deleted or modified to a smaller number.

26. The detection method of claim 23, wherein said further detection is performed when the actual number of read-write of the consumable cassette is zero.

27. The detection method of claim 13, wherein the test information comprises a threshold for insufficient sampling volume, and
wherein determining, according to the test information, whether the consumable is in compliance with the detection requirement comprises:
determining whether the sampling volume of the consumable is insufficient by comparing a calculated value of a pre-scanned data of the consumable by the detection instrument with the threshold for insufficient sampling volume;
displaying a message indicating that the sampling volume of the consumable is insufficient and terminating the detection when the sampling volume of the consumable is insufficient; and
performing a further detection when the sampling volume of the consumable is sufficient.

28. The detection method of claim 27, wherein the calculated value of the pre-scanned data is an intensity of a signal.

29. The detection method of claim 28, wherein the signal is a fluorescent signal.

30. The detection method of claim 28, wherein the signal is a non-fluorescent signal.

31. The detection method of claim 30, wherein the non-fluorescent signal is a light absorption signal, an electrical signal, a magnetic signal or a color signal.

32. The detection method of claim 27, wherein the detection instrument is configured to perform a sampling at one position of the detection side of the consumable to obtain the calculated value of the pre-scanned data.

33. The detection method of claim 32, wherein the detection instrument is configured to perform a sampling at one position of the detection side of the consumable at multiple time points, and average multiple sampling values to obtain the calculated value of the pre-scanned data.

34. The detection method of claim 27, wherein the detection instrument is configured to perform a sampling at multiple positions of the detection side of the consumable, and average multiple sampling values to obtain the calculated value of the pre-scanned data.

35. The detection method of claim 34, wherein the detection instrument is configured to perform a sampling at multiple positions of the detection side of the consumable at multiple time points, and average multiple sampling values to obtain the calculated value of the pre-scanned data.

36. The detection method of claim 13, wherein the test information comprises a threshold for reaction over time failure, and
wherein determining, according to the test information, whether the consumable is in compliance with the detection requirement comprises:
determining whether the consumable reaction time is unreasonably long by comparing a calculated value of a pre-scanned data of the consumable by the detection instrument with the threshold for reaction over time failure;
displaying a message indicating that the consumable reaction time is unreasonably long and terminating the detection when the consumable reaction time is unreasonably long; and
performing a further detection when the consumable reaction time is not unreasonably long.

37. The detection method of claim 36, wherein the calculated value of the pre-scanned data is an intensity of a signal.

38. The detection method of claim 37, wherein the signal is a fluorescent signal.

39. The detection method of claim 37, wherein the signal is a non-fluorescent signal.

40. The detection method of claim 39, wherein the non-fluorescent signal is a light absorption signal, an electrical signal, a magnetic signal or a color signal.

41. The detection method of claim 36, wherein the detection instrument is configured to perform a sampling at one position of the detection side of the consumable to obtain the calculated value of the pre-scanned data.

42. The detection method of claim 41, wherein the detection instrument is configured to perform a sampling at one position of the detection side of the consumable at multiple time points, and average multiple sampling values to obtain the calculated value of the pre-scanned data.

43. The detection method of claim 36, wherein the detection instrument is configured to perform a sampling at multiple positions of the detection side of the consumable, and average multiple sampling values to obtain the calculated value of the pre-scanned data.

44. The detection method of claim 43, wherein the detection instrument is configured to perform a sampling at multiple positions of the detection side of the consumable at multiple time points, and average multiple sampling values to obtain the calculated value of the pre-scanned data.

45. The detection method of claim 13, wherein the test information comprises a threshold for consumable failure, and
wherein determining, according to the test information, whether the consumable is in compliance with the detection requirement comprises:
determining whether the consumable fails by comparing a calculated value of a control line data scanned by the detection instrument with the threshold for consumable failure;
displaying a message indicating that the consumable fails and terminating the detection when the consumable fails, and
calculating and displaying a measurement result when the consumable does not fail.

46. The detection method of claim 45, wherein the calculated value of the control line data is a peak height and/or peak area of a control line.

47. The detection method of claim 46, wherein the peak height and/or peak area of the control line is calculated from a signal intensity of control line measured by the detection instrument in combination with a center point position, a half-peak width and a baseline width of consumable control line contained in the test information.

48. The detection method of claim 13, wherein the detection result comprises a single signal or a set of signals.

49. The detection method of claim 13, wherein the detection result is calculated from a test line signal intensity of the consumable measured by the detection instrument in combination with a center point position a half-peak width and a baseline width of consumable test line contained in the test information.

50. The detection method of claim 13, wherein a measurement result and a gear level of measurement result of target object are calculated based on the detection result in combination with a calibration curve, a correction parameter and a critical value of result gear level contained in the test information.

51. The detection method of claim 13, further comprising updating the test information stored in the circuit board of the consumable cassette.

52. A consumable cassette, comprising a detection side provided with a consumable, wherein the consumable cassette is provided with a circuit board carrying a test information.

53. The consumable cassette of claim 52, wherein the circuit board comprises a chip for storing the test information.

54. The consumable cassette of claim 52, wherein the test information comprises one or more of an anti-counterfeiting code, a category of detection, a consumable number, a consumable batch number, date of production, expiration date, time of first read/write, number of read/write operations, a limit for number of read-write, a limit for consumable reaction time, a threshold for insufficient sampling volume, name of test line, name of control line, a parameter of signal pre-processing, a threshold for reaction over time failure, a threshold for consumable failure, a calibration curve, a correction parameter, a critical value of result gear level, and an unit of testing.

55. The consumable cassette of claim 54, wherein the parameter of signal pre-processing comprises one or more of a center point position of test line, a center point position of control line, a half-peak width and a baseline width.

56. The consumable cassette of claim 54, wherein the calibration curve comprises a type of curve and/or a parameter of curve.

57. A detection instrument, comprising:
a reading device configured to read a test information from a consumable cassette, wherein the consumable cassette is provided with a consumable and a circuit board carrying the test information; and
one or more processors configured collectively or individually to:
determine, according to the test information, whether the consumable is in compliance with a detection requirement; and
perform a detecting on the consumable to obtain a detection result when the consumable is in compliance with the detection requirement.

58. The detection instrument of claim 57, wherein the one or more processors are further configured to:
determine whether the consumable cassette is correctly installed;
read the test information from the circuit board when the consumable cassette is correctly installed; and
determine whether the consumable is in compliance with the detection requirement according to the test information.

59. The detection instrument of claim 57, wherein the one or more processors are further configured to:
determine the consumable is readable when the test information is able to be read from the circuit board and data is able to be stored into the circuit board;
display a message indicating that the consumable is not readable and terminate the detection when the consumable is not readable; and
perform a further detection when the consumable is readable.

60. The detection instrument of claim 57, wherein the test information comprises an anti-counterfeiting code, and
wherein the one or more processors are further configured to:
determine, according to the anti-counterfeiting code, whether the consumable is a counterfeit;
display a message indicating that the consumable is a counterfeit and terminate the detection when the consumable is a counterfeit; and
perform a further detection when the consumable is not a counterfeit.

61. The detection instrument of claim 57, wherein the test information comprises a category of detection, a consumable number and a consumable batch number, and
wherein the one or more processors are further configured to:
determine, according to the category of detection, the consumable number and the consumable batch number, whether the consumable is a product being recalled;
display a message indicating that the consumable is a product being recalled and terminate the detection when the consumable is a product being recalled; and
perform a further detection when the consumable is not a product being recalled.

62. The detection instrument of claim 57, wherein the test information comprises date of production and expiration date, and
wherein the one or more processors are further configured to:
determine, according to the date of production, the expiration date and a current time of the detection instrument, whether the consumable is expired;
display a message indicating that the consumable is expired and terminate the detection when the consumable is expired; and
perform a further detection when the consumable is not expired.

63. The detection instrument of claim 57, wherein the test information comprises a limit for consumable reaction time, and
wherein the one or more processors are further configured to:
determine, by comparing an actual reaction time of the consumable and the limit for consumable reaction time, whether a consumable reaction time is beyond limit;
display a message indicating that the consumable reaction is beyond limit and terminate the detection when the consumable reaction time is beyond limit; and
perform a further detection when the consumable reaction time is not beyond limit.

64. The detection instrument of claim 63, wherein the detection instrument is configured to, once the consumable cassette is inserted into the detection instrument, write into the circuit board of the consumable cassette a current system time as an actual reaction start time of the consumable.

65. The detection instrument of claim 64, wherein the actual reaction start time of the consumable is not deleted or modified.

66. The detection instrument of claim 64, wherein the actual reaction time of the consumable is determined from the actual reaction start time of the consumable and the current system time.

67. The detection instrument of claim 57, wherein the test information comprises a limit of number of read-write times, and
wherein the one or more processors are further configured to:
determine whether the consumable is overused by comparing an actual number of read-write of the consumable cassette with the limit for number of read-write, said actual number of read-write of the consumable cassette being stored in the circuit board of the consumable cassette;
display a message indicating that the consumable is overused when the consumable is overused; and
perform a further detection when the consumable is not overused.

68. The detection instrument of claim 67, wherein the actual number of read-write of the consumable cassette is increased by one each time the consumable cassette is inserted into the detection instrument.

69. The detection instrument of claim 67, wherein the actual number of read-write times of the consumable cassette is not deleted or modified to a smaller number.

70. The detection instrument of claim 67, wherein the one or more processors are further configured to perform the further detection when the actual number of read-write times of the consumable cassette is zero.

71. The detection instrument of claim 57, wherein the test information comprises a threshold for insufficient sampling volume, and wherein the one or more processors are further configured to:
determine whether the sampling volume of the consumable is insufficient by comparing a calculated value of a pre-scanned data of the consumable by the detection instrument with the threshold for insufficient sampling volume;
display a message indicating that the sampling volume of the consumable is insufficient and terminate the detection when the sampling volume of the consumable is insufficient; and
perform a further detection when the sampling volume of the consumable is sufficient.

72. The detection instrument of claim 71, wherein the calculated value of the pre-scanned data is an intensity of a signal.

73. The detection instrument of claim 72, wherein the signal is a fluorescent signal.

74. The detection instrument of claim 72, wherein the signal is a non-fluorescent signal.

75. The detection instrument of claim 74, wherein the non-fluorescent signal is a light absorption signal, an electrical signal, a magnetic signal or a color signal.

76. The detection instrument of claim 71, wherein the one or more processors are further configured to direct the detection instrument to perform a sampling at one position of a detection side of the consumable to obtain the calculated value of the pre-scanned data.

77. The detection instrument of claim 76, wherein the one or more processors are further configured to: direct the detection instrument to perform a sampling at one position of the detection side of the consumable at multiple time points, and average multiple sampling values to obtain the calculated value of the pre-scanned data.

78. The detection instrument of claim 71, wherein the one or more processors are further configured to: direct the detection instrument to perform a sampling at multiple positions of the detection side of the consumable, and average multiple sampling values to obtain the calculated value of the pre-scanned data.

79. The detection instrument of claim 78, wherein the one or more processors are further configured to: direct the detection instrument to perform sampling at multiple positions of the detection side of the consumable at multiple time points, and average multiple sampling values to obtain the calculated value of the pre-scanned data.

80. The detection instrument of claim 57, wherein the test information comprises a threshold for reaction over time failure, and
wherein the one or more processors are further configured to:
determine whether the consumable reaction time is unreasonably long by comparing a calculated value of a pre-scanned data of the consumable by the detection instrument with the threshold for reaction over time failure;
display a message indicating that the consumable reaction time is unreasonably long and terminate the detection when the consumable reaction time is unreasonably long; and
perform a further detection when the consumable reaction time is not unreasonably long.

81. The detection instrument of claim 80, wherein the calculated value of the pre-scanned data is an intensity of a signal.

82. The detection instrument of claim 81, wherein the signal is a fluorescent signal.

83. The detection instrument of claim 81, wherein the signal is a non-fluorescent signal.

84. The detection instrument of claim 83, wherein the non-fluorescent signal is a light absorption signal, an electrical signal, a magnetic signal or a color signal.

85. The detection instrument of claim 80, wherein the one or more processors are further configured to direct the detection instrument to perform a sampling at one position of a detection side of the consumable to obtain the calculated value of the pre-scanned data.

86. The detection instrument of claim 85, wherein the one or more processors are further configured to: direct the detection instrument to perform sampling at one position of the detection side of the consumable at multiple time points, and average multiple sampling values to obtain the calculated value of the pre-scanned data.

87. The detection instrument of claim 80, wherein the one or more processors are further configured to: direct the detection instrument to perform sampling at multiple positions of the detection side of the consumable, and average multiple sampling values to obtain the calculated value of the pre-scanned data.

88. The detection instrument of claim 87, wherein the one or more processors are further configured to: direct the detection instrument to perform sampling at multiple positions of the detection side of the consumable at multiple time points, and average multiple sampling values to obtain the calculated value of the pre-scanned data.

89. The detection instrument of claim 57, wherein the test information comprises a threshold for consumable failure, and
wherein the one or more processors are further configured to:
determine whether the consumable fails by comparing a calculated value of a control line data scanned by the detection instrument with the threshold for consumable failure;
display a message indicating that the consumable fails and terminating the detection when the consumable fails, and
calculate and display a measurement result when the consumable does not fail.

90. The detection instrument of claim 89, wherein the calculated value of the control line data is a peak height and/or peak area of a control line.

91. The detection instrument of claim 57, wherein the peak height and/or peak area of the control line is calculated from a signal intensity of control line measured by the detection instrument in combination with a center point position, a half-peak width and a baseline width of consumable control line contained in the test information.

92. The detection instrument of claim 57, wherein the detection result comprises a single signal or a set of signals.

93. The detection instrument of claim 57, wherein the one or more processors are further configured to: calculate the detection result from a test line signal intensity of the consumable measured by the detection instrument in combination with a center point position a half-peak width and a baseline width of consumable test line contained in the test information.

94. The detection instrument of claim 57, wherein the one or more processors are further configured to: calculate a measurement result and a gear level of measurement result of target object based on the detection result in combination with a calibration curve, a correction parameter and a critical value of result gear level contained in the test information.

95. The detection instrument of claim 57, wherein the one or more processors are further configured to update the test information stored in the circuit board of the consumable cassette.
